# EUROPEAN PATENT SPECIFICATION

(11) **EP 2 214 728 B1**
(45) Date of publication and mention of the grant of the patent: **27.06.2012**
(21) Application number: 08806234.4
(22) Date of filing: 10.09.2008
(51) Int. Cl.: A61L 15/18, A61L 15/60, A61L 15/46, A61L 15/42, A61F 13/02

(54) **WOUND DRESSING WITH APERTURED COVER SHEET**
WUNDVERBAND MIT ABDECKTEIL MIT ÖFFNUNGEN
PANSEMENT MUNI D'UNE FEUILLE DE COUVERTURE QUI COMPORTE DES OUVERTURES

(30) Priority: 11.09.2007 GB 0717698
(43) Date of publication of application: 11.08.2010
(73) Proprietor: Systagenix Wound Management IP Co. BV., 1015 CS Amsterdam (NL)
(72) Inventor: CLARK, Rachael, Louise, Skipton BD23 2DH (GB); STEDMAN, Margaret, Harrogate North Yorkshire HG2 7RL (GB); STEPHENS, Sally, Skipton BD23 2JX (GB); BAYLIFF, Simon, North Yorkshire BD23 6AH (GB)
(74) Representative: James, Anthony Christopher W.P.
(86) International application number: PCT/GB2008/003075
(87) International publication number: WO 2009/034322

(56) References cited:
- EP-A1- 0 097 517
- WO-A1-02/43743
- WO-A1-02/062403
- WO-A1-03/045294
- WO-A1-03/053484
- WO-A1-2004/024197
- WO-A1-2004/112852
- GB-A- 2 392 836
- GB-A- 2 393 655
- US-A- 4 715 857
- US-A1- 2005 054 998

## Description

The present invention relates to a layered wound dressing comprising an antimicrobial absorbent layer and an apertured sheet covering the antimicrobial absorbent layer.

It is known that the adherency of wound dressings based on nonwoven textile absorbent layers can be reduced by providing a suitable apertured top sheet (wound contacting sheet) over the textile layer in the wound dressing. The top sheet reduces the tendency of the fibrous absorbent layer to adhere to the surface of a wound in use.

For example, GB-A-2074029 describes wound dressings having an absorbent layer of fibrous material and a top sheet of perforated polytetrafluoroethylene (PTFE) film covering the absorbent layer. The PTFE film provides a non-adherent wound contacting layer for the dressing.

GB-A-2085305 describes wound dressings in which a fibrous absorbent layer is entirely enclosed by a cellular plastics film having perforations of diameter 0.2mm to 0.8mm which have been produced by passing electrical discharges therethrough. The small apertures are said to result in low adherency of the wound contacting surface.

GB-A-1526778 describes wound dressings comprising an absorbent layer and a top sheet over the absorbent layer, wherein the top sheet is a plastic film having perforations that are tapered in cross-section, whereby the perforations promote the passage of wound fluid into the absorbent layer, but resist the flow of liquid from the absorbent layer back to the wound contacting surface.

EP-A-0275353 describes absorbent wound dressings comprising a top sheet formed of an elastomeric, soft, non-absorbent polyurethane film less than 50 micrometers thick, thermally bonded to a surface of an absorbent textile wound dressing, each perforation in the film having open areas equal to a circle having a diameter of 0.25 to 5mm, the perforations being present in sufficient number, and so distributed, as to provide an open area in the range from 5% to 25% of the total area of the film. The use of a soft, elastomeric film is said to reduce tissue damage by the film. The selection of aperture size and open area is said to provide the desired balance of absorbency, non-adherency and mechanical properties.

GB-A-2392836 describes wound dressings comprising an absorbent layer and a top sheet having apertures that are initially blocked by a material that can be broken down by certain enzymes in wound fluid. The presence of these enzymes in the wound selectively activates the release of active agents from the absorbent layer by breaking down the material in the apertures and thereby increasing the effective open area of the top sheet. The apertures make up from 0.1 to 50% of the area of the top sheet, and the mean area of each aperture is from about 0.01 to about 10mm².

The above art relates to wound dressings for the absorption of wound fluid. However, the use of a perforated top sheet potentially reduces the therapeutic effectiveness of more advanced wound dressings, in which the absorbent layer further comprises a therapeutic agent for delivery to the wound surface.

US-A-4715857 describes a wound dressing comprising a fibrous, silver-loaded charcoal cloth layer enclosed in an envelope of a perforated plastics film having an aperture size of 0.05 to 0.5mm. In such dressings, the use of such small perforations in the top sheet would be expected to inhibit the delivery of the therapeutic agent.

WO03/053584 describes wound dressing materials in the form of a nonwoven fabric made up of a mixture of hydrogel-forming absorbent fibers and non-absorbent textile fibers, in which some of the non-absorbent textile fibers are coated with metallic silver (Ag⁰) to give the dressings antimicrobial properties. Dressings of this type comprising calcium alginate, carboxymethylcellulose (CMC) hydrogel-forming fibers blended with silver-coated nylon fibers are commercially available under the Registered Trade Mark SILVERCEL. Adherency remains a problem with hydrogel-containing dressings of this type. Moreover, the silver-coated fibers have a dark color, and it is therefore desirable to avoid shedding of these fibers into the wound under treatment because of the undesirable appearance of the dark fibers in the wound.

EP-A-0097517 describes wound dressings having a foam core containing a silver salt (not metallic silver) and an apertured top sheet. The top sheet appears to be an open web, having aperture size 0.1-2.5mm and open area 35%-65%. The example of D1 has a web with 16 apertures per cm², having diameter 1.4mm, which implies about 40% open area.

WO-A-0345294 describes wound dressings having a hydrogel layer intermediate an apertured top sheet and an absorbent layer. The absorbent layer in these dressings may contain silver compounds, but the use of metallic silver is not disclosed. The top sheets have open area 10-25% and pore size 0.05-2mm, preferably 0.1-0.5mm.

WO-A-2004112852 describes dressings having a coating of metallic silver on an absorbent layer, and an apertured top sheet. The top sheet may have pore size 0.25-1.4mm and open area 15-60%. The example has apertures of size 0.4-0.7mm.

WO-A-02062403 describes sodium zirconium phosphate complexes of ionic silver dispersed preferably in a foam matrix.

WO-A-0243743 describes wound dressings containing silver salts that are stabilised against the action of light by complexation of the silver with a polymer.

WO-A-2004024197 describes freeze dried sponge materials based on oxidized regenerated cellulose and containing ionic silver complexed to the ORC. The specification mentions a liquid-permeable top sheet, but no details are given.

US-A-20050054998 describes absorbent articles, including wound dressings, having an absorbent layer and an apertured top sheet. The top sheet has open area preferably 4% to 30% and pore size preferably 0.01 to 2mm². No reason is given for this selection of aperture size and density, and there is no disclosure of silver in the absorbent layer.

GB-A-2393655 describes a wound dressing comprising an absorbent layer enclosed in an envelope that has a small number of apertures that are occluded with an erodible material before use. The area of the apertures is preferably 1% to 30%, more preferably 1% to 10%, and the aperture size is 1 to 400mm², preferably 10 to 100mm². The dressing may contain metallic silver.

It would therefore be desirable to provide wound dressings of this type, wherein the shedding of silver-coated fibers into the wound is prevented, but with minimum loss of antimicrobial effectiveness.

The present invention provides a wound dressing comprising an antimicrobial absorbent layer containing metallic silver, and an apertured sheet covering the antimicrobial absorbent layer, wherein the apertured sheet is formed from a liquid-impermeable sheet material having an array of open apertures therein and said wound dressing is sterile and packaged in a microorganism-impermeable container, characterized in that said apertures have a mean effective diameter of from 1mm to 2mm, and the percentage open area of the apertured sheet is from 17% to 25%.

The absorbent layer may comprise any of the materials conventionally used for absorbing wound fluids, serum or blood in the wound healing art, including gauzes, nonwoven fabrics, superabsorbents, hydrogels and mixtures thereof. Suitably, the absorbent layer comprises or consists essentially of a nonwoven textile fabric, for example a carded web of staple fibers. Suitably, the fabric comprises at least about 10wt.% of hydrogel-forming absorbent fibers based on the dry weight of the fabric, for example, the fabric comprises at least about 20wt.% of the hydrogel-forming fibers, for example from about 30wt.% to about 50wt.% of such fibers.

The term "hydrogel-forming fibers" refers to fibers that can absorb at least about twice their own weight of water, suitably at least about four times their own weight of water, to form a hydrogel. The fibers are normally insoluble in water. Suitable materials for the hydrogel-forming fibers include alginates, carboxymethylcelluloses, hydroxyethylcelluloses, polyacrylates, and hyaluronates. Preferred materials are calcium alginate and sodium carboxymethylcellulose and mixtures thereof.

Suitably, the fabric comprises at least about 10wt.% based on the dry weight of the fabric of substantially non-water-absorbent textile fibers, and suitably it comprises at least about 20wt.% of such fibers, for example from about 30wt.% to about 60wt.% of such fibers. Suitable non-absorbent textile fibers include polyamide fibers such as nylon fibers, polyolefin fibers, and viscose fibers.

Suitably, the absorbent layer is similar to those described in WO03/053584. That is to say, the absorbent layer comprises or consists essentially of a nonwoven fabric made up of a mixture of from 10wt.% to 90wt.% of hydrogel-forming absorbent fibers and from 90wt.% to 10wt.% of non-absorbent textile fibers, in which at least some of the non-absorbent textile fibers are coated with metallic silver (Ag⁰).

The term "fibers" herein generally refers to staple fibers, but it may refer to longer textile fibers. It does not refer to pulp fibers. In any case, the median length of the fibers used to form the fabric is generally at least about 10mm. Suitably, the amount of silver in the fabric is from 0.1% to 10wt.%, based on the dry weight of the fabric.

The absorbent layer contains silver as an antimicrobial agent.

The silver is metallic silver, suitably either colloidal metallic silver or a silver coating on one or more components of the absorbent layer. Suitably, the absorbent layer is a nonwoven textile layer wherein at least a fraction of the fibers in the absorbent fabric are coated with the silver to provide antimicrobial activity. Suitably, the silver coating is metallic silver applied to non-absorbent textile fibers of the fabric. The total amount of silver in the absorbent layer is typically from 0.5wt.% to 10wt.%, for example from 1wt.% to 5wt.% for metallic silver. These percentages are based on the dry weight of the absorbent layer.

The area of the absorbent layer is typically in the range of from 1 cm² to 200cm², more suitably from 4cm² to 150cm² The shape of the absorbent layer may for example be circular, elliptical, square, rectangular, or other polygonal. Suitably, the basis weight of the absorbent layer is in the range of 50-1000g/m², more suitably 100-500g/m². The uncompressed thickness of the absorbent layer is suitably in the range of from 0. 5mm to 10mm, more suitably 1.5 mm to 5mm. The free (uncompressed) liquid absorbency measured for physiological saline is suitably in the range of 5 to 100g/g at 25°C.

The thermoplastic film of the apertured cover sheet may be formed from substantially any thermoplastic film-forming polymer. Suitably, the polymer is conformable but not elastomeric. Suitably, the polymer is hydrophilic. Suitable polymers include, but are not limited to, polyethylene, polypropylene, polyester, polyamides such as nylons, fluoropolymers such as polyvinylidene fluoride (PVDF) or polytetrafluoroethylene

(PTFE), and mixtures thereof. The currently preferred film forming thermoplastic polymer is ethylene methyl acrylate (EMA).

The apertured cover sheet may be textured. The term "textured" indicates that the film is patterned in relief, for example, patterned with protruding ridges or nubbles, for example by embossing. The texturing renders the film less adherent to a wound bed. The ridges or nubbles may be rounded, and may project by 0.1 to 1.5 mm above the median plane of the film surface, for example by 0.2 to 1.0 mm above the median plane of the film.

In certain embodiments the cover sheet is both textured and perforated by means of mesh perforation. In this method, the film is supported on a reticulated mesh surface and heated to its softening temperature. Suction is then applied through the mesh, or air is blown onto the film above the mesh, which results in impression of the mesh into the film and the formation of perforations in the film at the interstices of the mesh. Mesh perforation techniques are described in more detail in US-A-3054148.

The cover sheet should be as thin as possible, consistent with the need for physical integrity during manufacture and use. Typically, the sheet has a basis weight of from 1 to 500g/m², for example from 10 to 200g/m². The apertures are open both before and during use. That is to say, they are not obstructed by a second material as described for example in GB-A-2392836.

As already noted, the size and density of the apertures in the top sheet is an important feature of the present invention. The apertures have an effective diameter of from 1mm to 2mm, and the percentage open area of the apertured sheet is from 17% to 25%. The term "mean effective diameter" refers to the diameter of a circle having equal area to the mean area of the apertures in the top sheet. The apertures may have non-circular shapes, but suitably substantially all of the apertures are circular. The apertures may be of uniform size, or there may be apertures of more than one size in the apertured sheet; for example, there may be a pattern of differently sized apertures. Not all of the apertures in the top sheet must have effective diameters in the specified range, so long as the mean effective diameter is in the specified range. However, suitably, at least about 50% of the apertures have effective diameters in the specified range, more suitably at least about 80%, and still more suitably substantially 100%. The term "percentage open area" refers to the percentage of the area of the cover sheet that is taken up by the open area of the apertures.

The mean effective diameter of the apertures is suitably from 1.2mm to 1.8mm. The percentage open area of the apertured sheet is suitably from 18% to 25%. Suitably, the mean center-to-center distance between adjacent apertures is from 2mm to 5mm, for example from 3mm to 5mm.

A suitable cover sheet has apertures with mean effective diameter from 1.1mm to 1.6mm and percentage open area from 19% to 25%. For example, the cover sheet may have apertures with mean effective diameter of about 1.3mm and percentage open area of about 22%.

The apertures may be any shape, but suitably they are substantially circular. The apertures are suitably substantially uniformly distributed over the top sheet, for example the apertures are suitably arranged in a regular array or pattern. Suitably, the cover sheet has from 5 to 50 apertures/cm², more suitably from 10 to 30 apertures/cm².

The apertured sheet material is positioned so that, in use, it lies between the wound contacting surface of the wound dressing and the absorbent layer. Suitably, the apertured sheet is the top sheet of the dressing, that is to say the material forms the wound contacting surface of the dressing. Suitably, the apertured sheet material is bonded directly to a wound facing surface of the absorbent layer by the application of heat and pressure to melt-bond the thermoplastic apertured sheet onto the absorbent layer. Apertured sheets according to the invention may be bonded in this way to both major surfaces of the absorbent layer.

The apertured sheet material can be formed into an envelope for the absorbent layer. The term "envelope" signifies that the front and back faces of the absorbent layer are substantially completely covered (substantially completely enclosed) by the apertured sheet. For example, two sheets of the apertured sheet may be located above and below the absorbent layer substantially covering the top and bottom faces of the absorbent layer, the two sheets being bonded along two or more edges to form the envelope. In certain embodiments the envelope is formed from a single piece of the apertured sheet that has been folded around the absorbent layer so that opposed longitudinal edges of the sheet overlap, the overlapping edges being bonded together in the overlapping region, typically with hot melt adhesive or by heat bonding, to form the envelope. Such envelopes may be made by minor modification of conventional form-fill-seal equipment, as described further below.

The wound dressing may comprise a backing sheet extending over the absorbent layer opposite to the wound facing side of the absorbent layer. Suitably, the backing sheet is larger than the absorbent layer such that a marginal region of width 1mm to 50mm, suitably 5mm to 20mm extends around the active layer to form a so-called island dressing. In such cases, the backing sheet is suitably coated with a pressure sensitive medical grade adhesive in at least its marginal region.

Suitably, the backing sheet is substantially liquid-impermeable. The backing sheet is suitably semipermeable. That is to say, the backing sheet is suitably permeable to water vapour, but not permeable to liquid water or wound exudate. Suitably, the backing sheet is also microorganism-impermeable. Suitable polymers for forming the backing sheet are well known in the wound dressing art.

The dressing may comprise further layers. For example, these layers may comprise further absorbent layers.

The wound facing surface of the dressing may be protected by a removable cover sheet. The cover sheet is normally formed from flexible thermoplastic material. Suitable materials include polyesters and polyolefins. Suitably, the adhesive- facing surface of the cover sheet is a release surface. That is to say, a surface that is only weakly adherent to the active layer and the adhesive on the backing sheet to assist peeling of the adhesive layer from the cover sheet. For example, the cover sheet may be formed from a non-adherent plastic such as a fluoropolymer, or it may be provided with a release coating such as a silicone or fluoropolymer release coating.

The wound dressing according to the present invention is sterile and packaged in a microorganism-impermeable container.

Specific embodiments of the present invention will now be described further, by way of example, with reference to the accompanying drawings, in which:
Fig. 1 shows a perspective view of a dressing according to the invention;
Fig. 2 shows schematic drawings of the aperture distribution in five different top sheets used for testing;
Fig. 3 shows a plot of measured silver release after 24 hours versus aperture size and open area of the top sheet for a dressing containing metallic silver;
Fig. 4 shows a contour plot of measured log10 bacterial reduction versus aperture size and open area of the top sheet for a dressing containing metallic silver

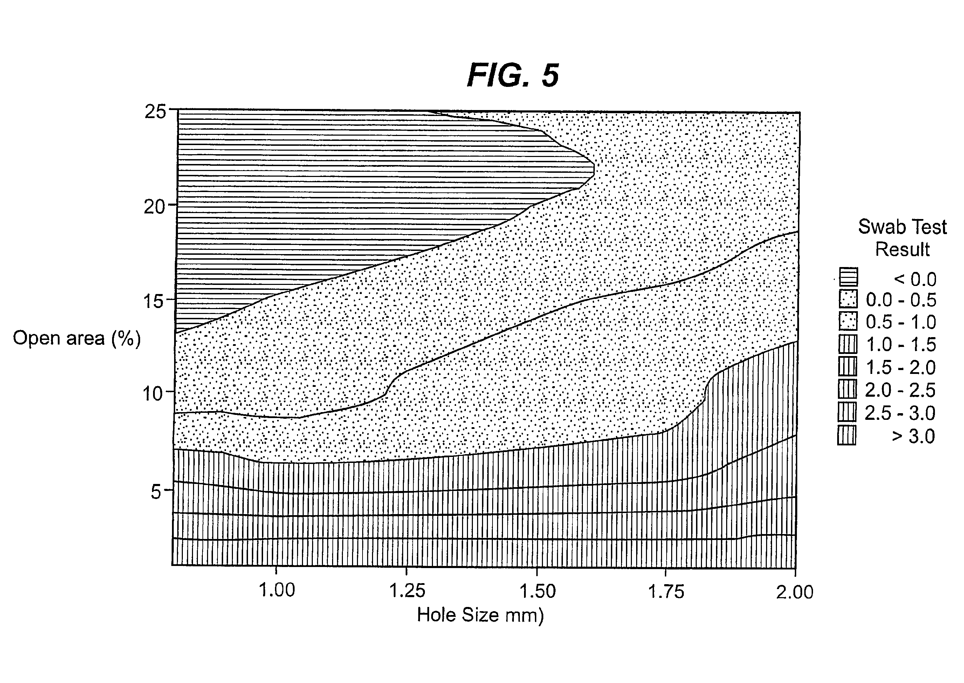

Fig. 5 shows a contour plot of swab test results in a three-day zone of inhibition test versus aperture size and open area of the top sheet for a dressing containing metallic silver
Fig. 6 shows a plot of measured silver release after 24 hours versus aperture size and open area of the top sheet for a reference dressing containing ionic silver; and
Fig. 7 shows a contour plot of measured log10 bacterial reduction versus aperture size and open area of the top sheet for a reference dressing containing ionic silver

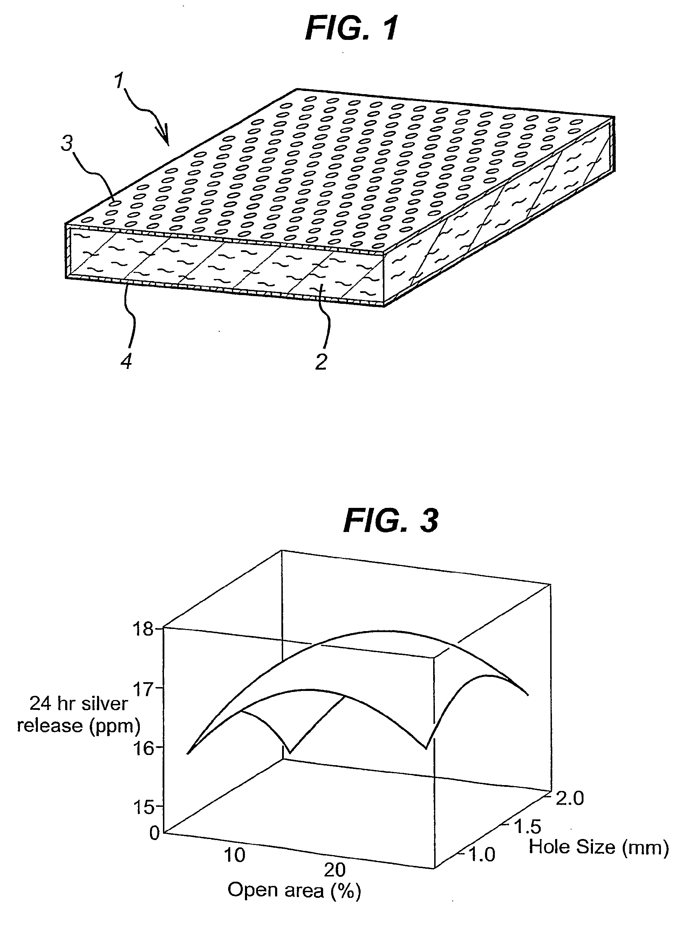

Referring to Fig. 1, the dressing 1 comprises an absorbent layer 2 containing metallic or ionic silver, having front and back sheets 3,4 of apertured EMA thermoplastic film bonded to the major surfaces thereof by application of heat and pressure.

### Example 1

Wound dressings according to the invention containing metallic silver were made with the structure shown in Fig. 1. The absorbent layer is a calcium alginate needled felt dressing incorporating silver-coated nylon fibers. The composition is as follows, by weight: calcium alginate and carboxymethyl cellulose (CMC) fibers 60% and silver coated nylon 40%. The basis weight of the fabric layer is about 150 g/m², and the uncompressed thickness of the fabric layer is about 2 mm. The total silver content of the fabric is about 8 wt.%. The fabric layer is commercially available from Johnson & Johnson under the Registered Trade Mark SILVERCEL.

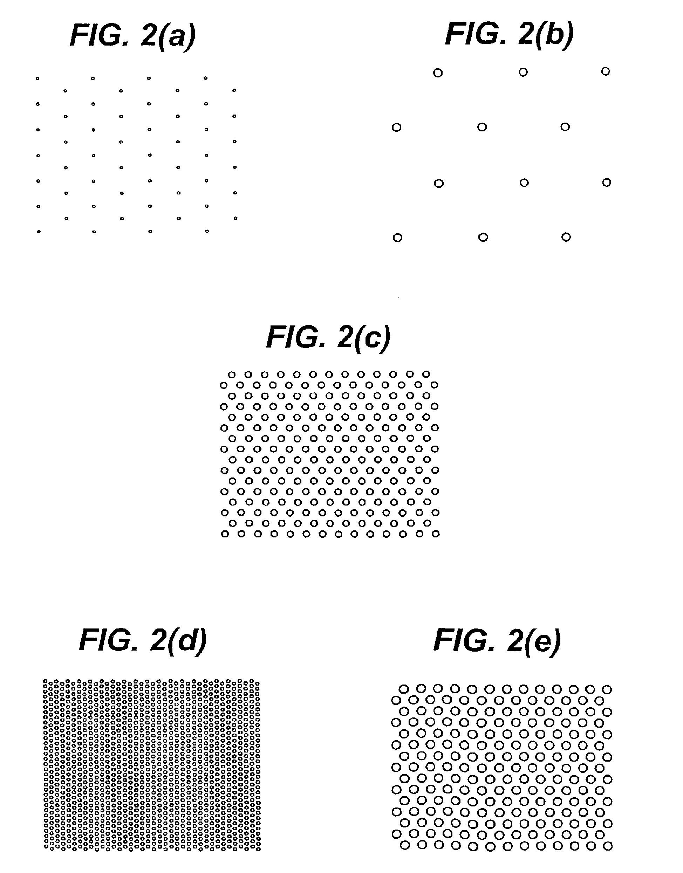

Dressings of this type were evaluated having top sheets with a range of hole sizes and percentage open areas, some of which are shown schematically in Fig.2. Specifically,

Fig. 2(a) represents 0.8mm hole size, 1% open area; Fig. 2(b) represents 2mm hole size, 1% open area; Fig. 2(c) represents 1.4mm hole size, 13% open area; Fig. 2(d) represents 0.8mm hole size, 25% open area; and Fig. 2(e) represents 2mm hole size, 25% open area.

A total of 15 dressings of this type having differently perforated top sheets were tested. Hole sizes tested were, 0.8, 1.4 and 2.0mm diameter, and percent open areas tested were 1%, 7%, 13%, 19% and 25%.

### Example 2 (Reference Example)

Wound dressings according to the invention containing ionic silver were made with the structure shown in Fig. 1. The absorbent layer is a calcium alginate felt dressing incorporating an ionic silver complex. The composition is as follows, calcium alginate, carboxymethyl cellulose (CMC) fibers and silver sodium hydrogen zirconium phosphate. The total silver content of the fabric is about 0.5 wt.%. The fabric layer is commercially available from Laboratories Urgo, under the Registered Trade Mark Urgosorb Silver.

A total of 5 dressings of this type having differently perforated top sheets were tested. Hole sizes tested were, 0.8, 1.4 and 2.0mm diameter, and percent open areas tested were 1%, 13%, and 25%.

### Procedure 1 - Silver Release

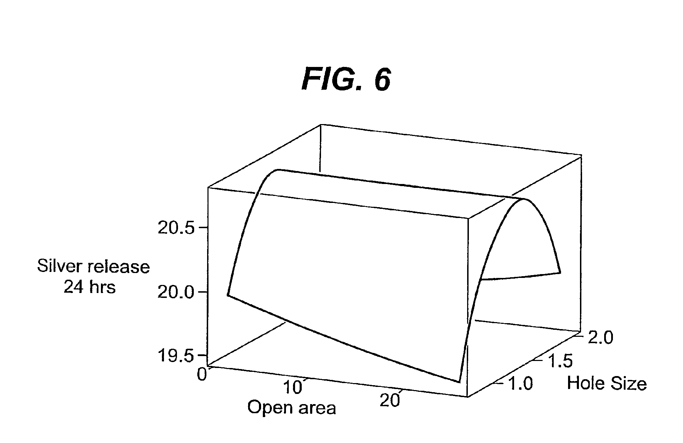

Samples of the dressings of Examples 1 and 2 were immersed into a solution of simulated wound fluid (SWF), 0.013M Calcium Chloride, 0.2M Sodium Chloride and 0.04M Tris containing 2% Bovine Albumin at pH 7.5. Samples were gently agitated in an amount of SWF equivalent to 5ml / 2.5 x 2.5cm². At a specific time point, for example 24 hours, a sample of fluid was removed and diluted in SWF and analysed against a silver standard Analysis was performed against a silver standard curve prepared in SWF using the Perkin Elmer Analyst 200 Atomic Absorption Spectrometer. Standards of known concentration were prepared in the same SWF as used for the samples. Specimen data for silver release in ppm are shown in Table 1 and are represented graphically in Figs. 3 and 6.

**Table 1: Silver release - Metallic Silver Dressings**

| | | **Percent open area** | | | | |
|---|---|---|---|---|---|---|
| | | **1** | **7** | **13** | **19** | **25** |
| **Hole size (mm)** | 0.8 | 16.3+/-26 | 16.2+/1.1 | 16.7+/-0.8 | 16.7+/-0.6 | 16.4+/-1.3 |
| | 1.4 | 16.4+/-1.3 | 18.0+/-1.0 | 17.7+/-1.4 | 17.4+/-1.4 | 17.4+/-1.1 |
| | 2 | 15.0+/-0.7 | 15.6+/-1.4 | 16.5+/-1.2 | 17.1+/-1.8 | 15.9+/-1.6 |

**Table 2: Silver release - Ionic Silver Dressings (Reference example)**

| | | **Percent open area** | | |
|---|---|---|---|---|
| | | **1** | **13** | **25** |
| **Hole size (mm)** | 0.8 | 19.6 +/- 0.37 | 20.41 +/- 0.88 | 19.21 +/- 0.53 |
| | 1.4 | 20.75 +/- 0.33 | 20.04 +/- 0.18 | 20.38 +/-1.38 |
| | 2 | 20.00 +/- 0.83 | 18.73 +/- 0.61 | 20.5 +/- 0.95 |

It can be seen that there is a maximum in the silver release from the metallic silver dressings at a hole size of 1.4mm and percentage open area 13%. At larger hole sizes and larger percentage open area the silver release is unexpectedly lower. The ionic silver dressings showed a maximum silver release at hole size of 1.4mm, apparently independent of open area in the samples tested.

### Procedure 2 - Inactivation of Bacteria in Suspension

Testing was performed using common wound pathogens, *Staphylococcus aureus* and *Pseudomonas aeruginosa.* The test dressings were immersed in a quantified bacterial suspension and then sampled to determine the numbers of challenge bacteria killed over a 120 minute test period. The log₁₀ bacterial inactivation was calculated.

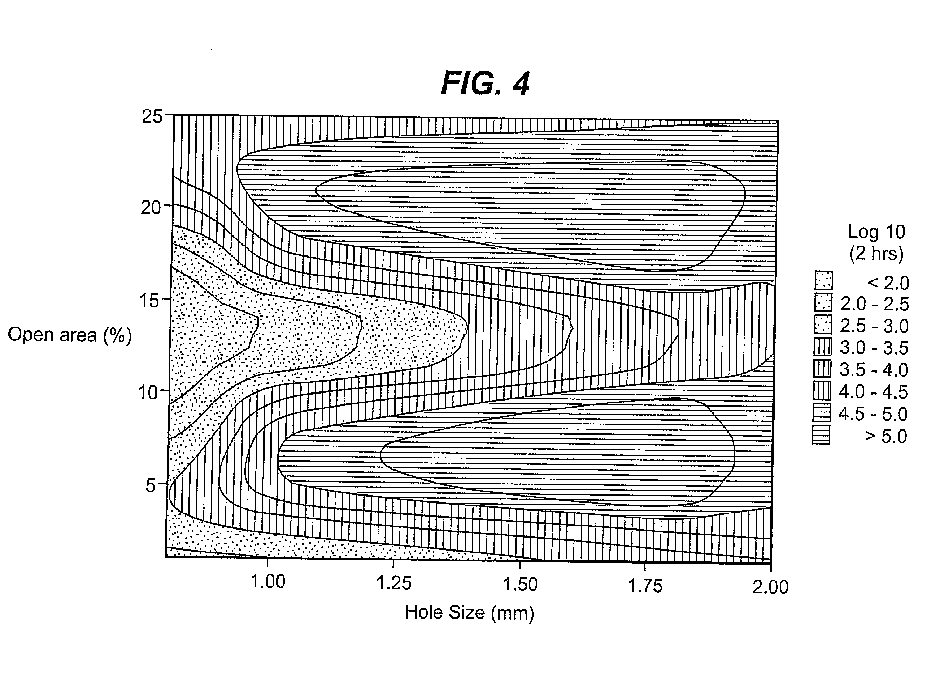

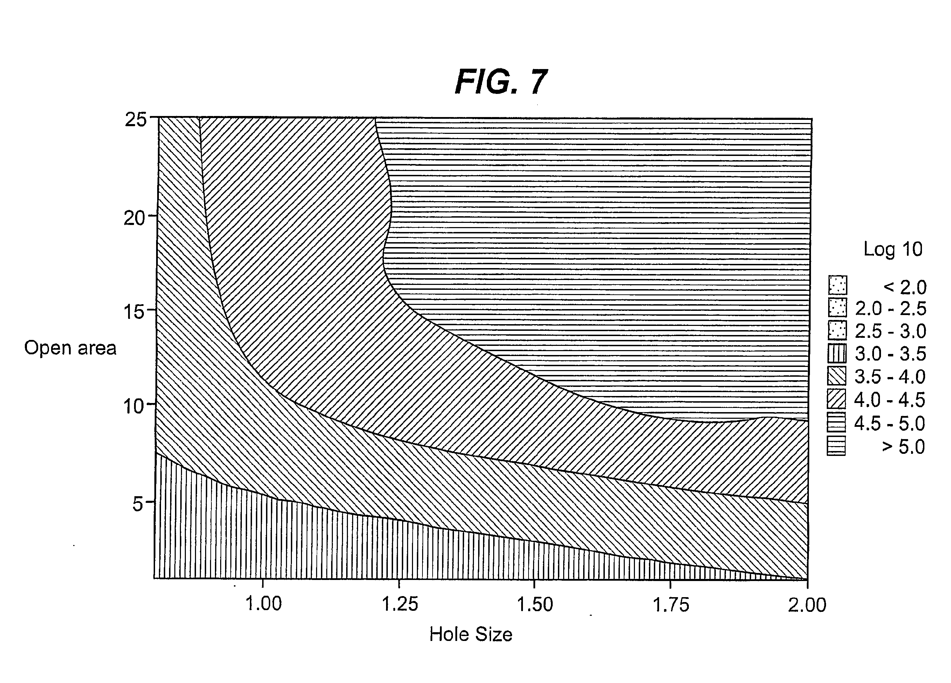

The results are shown graphically in Figs. 4 and 7 as contour plots. For the metallic silver dressings of Fig. 4, it can be seen that the log₁₀ bacterial inactivation increases sharply at hole sizes above about 0.8mm, and shows two distinct maxima at percentage open areas about 7% and about 20%. For the ionic silver reference dressings of Fig. 7, it can be seen that the log₁₀ bacterial inactivation increases sharply at hole sizes above about 1.0mm, and at open area above about 7%.

### Procedure 3 - Three Day Zone of Inhibition Test and Swab Test

Testing was performed using agar plates inoculated with *Staphylococcus aureus.* Silver preferentially acts upon gram-negative bacteria (*Pseudomonas aeruginosa*) over gram-positive bacteria. (*Staphylococcus aureus*), and therefore *Staphylococcus aureus* was considered a more demanding test organism for the dressings of the invention. Dressings were transferred onto freshly inoculated agar plates on a daily basis for three days. The zone of inhibition was calculated by measuring the distance from the edge of the dressing to the edge of the clear zone surrounding the dressing. These measurements were made daily, prior to dressing transfer in order to determine the antimicrobial activity of the wound dressings, and whether this activity was sustained over three days. The mean ± standard deviation was calculated for the zone of inhibition.

In addition, the ability of the dressing to prevent the growth of bacteria beneath the dressing was determined by taking a swab sample from the agar surface in direct contact with the dressing material and testing the swab for the presence of the bacteria.

The zone of inhibition (in mm) and swab results from dressings tested against *Staphylococcus aureus* are given in Tables 3 and 4. Positive results indicate growth of bacteria from swabs taken from the agar surface. One positive (1⁺) swab result is within the normal range of currently marketed anti-microbial dressings, therefore acceptable. Greater than one positive result upon swabbing is not acceptable for this test method. Ideally, no positive swab results ("Negative swab") results should be obtained.

**Table 3: Swab results - Metallic Silver**

| Open Area (%) | Hole Size (mm) | | |
|---|---|---|---|
| | 0.8 | 1.4 | 2.0 |
| 1 | 3.13 ± 0.53 | - | 3.11 ± 0.44 |
| | 3 + swab | | 3 + swab |
| 7 | - | 3.21 ± 0.36 | - |
| | | 1 + swab | |
| 13 | 3.13 ± 0.23 | - | 3.61 ± 0.64 |
| | Negative swab | | 1 + swab |
| 19 | - | 3.58 ± 0.34 | - |
| | | Negative swab | |
| 25 | 2.82±0.71 | - | 3.3 ± 0.40 |
| | Negative swab | | Negative swab |

**Table 4: Swab results - Ionic Silver (Reference example)**

| Open Area (%) | Hole Size (mm) | | |
|---|---|---|---|
| | 0.8 | 1.4 | 2.0 |
| I | 2.24 ± 0.71 | - | 2.83 ± 0.47 |
| | 2 + swab | | 3 + swab |
| 13 | - | 4.17 ± 0.45 | - |
| | | 2 + swab | |
| 25 | 4.89± 0.41 | - | 4.96 ± 0.56 |
| | Negative swab | | Negative swab |

It can be seen from the data that there is an optimal combination of aperture size and density for silver release and antimicrobial properties of the dressings.

The above embodiments have been described for the purpose of illustration only. Many other embodiments falling within the scope of the present invention will be apparent to the skilled reader.

## Claims

1. A wound dressing comprising an antimicrobial absorbent layer containing metallic silver, and an apertured sheet covering the antimicrobial absorbent layer, wherein the apertured sheet is formed from a liquid-impermeable sheet material having an array of open apertures therein and said wound dressing is sterile and packaged in a microorganism-impermeable container, **characterized in that** said apertures have a mean effective diameter of from 1mm to 2mm, and the percentage open area of the apertured sheet is from 17% to 25%.

2. A wound dressing according to claim 1, wherein the absorbent layer comprises or consists essentially of a nonwoven textile fabric comprising at least about 10wt.% of hydrogel-forming absorbent fibers based on the dry weight of the fabric.

3. A wound dressing according to any preceding claim, wherein the absorbent layer comprises a nonwoven fabric made up of a mixture of from 10wt.% to 90wt.% of hydrogel-forming absorbent fibers and from 90wt.% to 10wt.% of non-absorbent textile fibers, in which at least some of the non-absorbent textile fibers are coated with metallic silver (Ag⁰).

4. A wound dressing according to any preceding claim, wherein the apertured sheet material is melt-bonded directly to a wound facing surface of the absorbent layer.

5. A wound dressing according to any preceding claim, wherein the apertured sheet has apertures with mean effective diameter from 1.1mm to 1.6mm and percentage open area from 19% to 25%.

6. A wound dressing according to any preceding claim, wherein the total amount of silver in the absorbent layer is from 0.5wt.% to 10wt.% based on the dry weight of the absorbent layer.

7. A wound dressing according to claim 6, wherein the total amount of silver in the absorbent layer is from 1wt.% to 5wt.% based on the dry weight of the absorbent layer.

8. A wound dressing according to any preceding claim, wherein the apertured sheet has from 5 to 50 apertures/cm².

9. A wound dressing according to claim 8, wherein the apertured sheet has from 10 to 30 apertures/cm².

## Patentansprüche

1. Wundverband, umfassend eine antimikrobielle Absorbensschicht, die metallisches Silber enthält, und eine Folie mit öffnungen, die die antimikrobielle Absorbensschicht bedeckt, wobei die Folie mit Öffnungen aus einem flüssigkeitsundurchlässigen Folienmaterial gebildet ist, das ein Feld von offenen Öffnungen aufweist, und der Wundverband steril und in einem Mikroorganismen-undurchlässigen Behälter verpackt ist, **dadurch gekennzeichnet, dass** die Öffnungen einen mittleren effektiven Durchmesser von 1 mm bis 2 mm aufweisen und die prozentuelle offene Fläche der Folie mit Öffnungen von 17 % bis 25 % beträgt.

2. Wundverband gemäß Anspruch 1, wobei die Absorbensschicht ein Textilvlies umfasst oder im Wesentlichen daraus besteht, umfassend wenigstens etwa 10 Gew.-% Hydrogel-bildende Absorbensfasern, bezogen auf das Trockengewicht des Gewebes.

3. Wundverband gemäß einem der vorstehenden Ansprüche, wobei die Absorbensschicht ein Vlies umfasst, hergestellt aus einem Gemisch von 10 Gew.-% bis 90 Gew.-% Hydrogel-bildenden Absorbensfasern und von 90 Gew.-% bis 10 Gew.-% nicht-Absorbens-Textilfasern, wobei wenigstens manche der nicht-Absorbens-Textilfasern mit metallischem Silber (Ag⁰) beschichtet sind.

4. Wundverband gemäß einem der vorstehenden Ansprüche, wobei das Material der Folie mit Öffnungen direkt an eine wundseitige Oberfläche der Absorbensschicht schmelzgebunden ist.

5. Wundverband gemäß einem der vorstehenden Ansprüche, wobei die Folie mit Öffnungen Öffnungen mit einem mittleren effektiven Durchmesser von 1,1 mm bis 1,6 mm aufweist und die prozentuelle offene Fläche von 19 % bis 25 % beträgt.

6. Wundverband gemäß einem der vorstehenden Ansprüche, wobei die Gesamtmenge an Silber in der Absorbensschicht von 0,5 Gew.-% bis 10 Gew.-%, bezogen auf das Trockengewicht der Absorbensschicht, beträgt.

7. Wundverband gemäß Anspruch 6, wobei die Gesamtmenge an Silber in der Absorbensschicht von 1 Gew.-% bis 5 Gew.-%, bezogen auf das Trockengewicht der Absorbensschicht, beträgt.

8. Wundverband gemäß einem der vorstehenden Ansprüche, wobei die Folie mit Öffnungen 5 bis 50 Öffnungen/cm² aufweist.

9. Wundverband gemäß Anspruch 8, wobei die Folie mit Öffnungen 10 bis 30 Öffnungen/cm² aufweist.

## Revendications

1. Pansement comprenant une couche absorbante antimicrobienne contenant de l'argent métallique, et une feuille pourvue d'ouvertures recouvrant la couche absorbante antimicrobienne, la feuille pourvue d'ouvertures étant formée d'un matériau sous forme de feuille imperméable aux liquides contenant un ensemble d'ouvertures ouvertes et ledit pansement étant stérile et emballé dans un contenant imperméable aux micro-organismes, **caractérisé en ce que** lesdites ouvertures ont un diamètre effectif moyen de 1 mm à 2 mm, et la surface de vide de la feuille pourvue d'ouvertures représente un pourcentage de 17 % à 25 %.

2. Pansement selon la revendication 1, dans lequel la couche absorbante comprend ou consiste essentiellement en un textile non tissé comprenant au moins environ 10 % en poids de fibres absorbantes formant un hydrogel relativement au poids sec du textile.

3. Pansement selon l'une quelconque des revendications précédentes, dans lequel la couche absorbante comprend un tissu non tissé constitué d'un mélange de 10 % en poids à 90 % en poids de fibres absorbantes formant un hydrogel et de 90 % en poids à 10 % en poids de fibres textiles non absorbantes, au moins certaines des fibres textiles non absorbantes étant revêtues d'argent métallique (Ag⁰).

4. Pansement selon l'une quelconque des revendications précédentes, dans lequel le matériau constituant la feuille pourvue d'ouvertures est lié par fusion directement à une surface de la couche absorbante qui est orientée vers la plaie.

5. Pansement selon l'une quelconque des revendications précédentes, dans lequel la feuille pourvue d'ouvertures comporte des ouvertures ayant un diamètre effectif moyen de 1, mm à 1,6 mm et une surface de vide représentant un pourcentage de 19 % à 25 %.

6. Pansement selon l'une quelconque des revendications précédentes, dans lequel la quantité totale d'argent dans la couche absorbante est de 0,5 % en poids à 10 % en poids relativement au poids sec de la couche absorbante.

7. Pansement selon la revendication 6, dans lequel la quantité totale d'argent dans la couche absorbante est de 1 % en poids à 5 % en poids relativement au poids sec de la couche absorbante.

8. Pansement selon l'une quelconque des revendications précédentes, dans lequel la feuille pourvue d'ouvertures comporte de 5 à 50 ouvertures/cm².

9. Pansement selon la revendication 8, dans lequel la feuille pourvue d'ouvertures comporte de 10 à 30 ouvertures/cm².
